# EUROPÄISCHE PATENTSCHRIFT

(11) **EP 1 039 928 B1**
(45) Veröffentlichungstag und Bekanntmachung des Hinweises auf die Patenterteilung: **02.03.2005**
(21) Anmeldenummer: 98966656.5
(22) Anmeldetag: 21.12.1998
(51) Int. Cl.: A61K 39/12

(54) **INTRA-NASALE GELBFIEBER-IMPFUNG**
INTRANASAL YELLOW FEVER VACCINATION
VACCINATION PAR VOIE INTRA-NASALE CONTRE LA FIEVRE JAUNE

(30) Priorität: 22.12.1997 DE 19757301
(43) Veröffentlichungstag der Anmeldung: 04.10.2000
(73) Patentinhaber: DEUTSCHES PRIMATENZENTRUM GESELLSCHAFT MBH, 37077 Göttingen (DE)
(72) Erfinder: NIEDRIG, Matthias, D-12203 Berlin (DE); STAHL-HENNIG, Christiane, D-37083 Göttingen (DE); HUNSMANN, Gerhard, D-37077 Göttingen (DE)
(74) Vertreter: Huber, Bernhard, Dipl.-Chem.
(86) Internationale Anmeldenummer: PCT/EP1998/008387
(87) Internationale Veröffentlichungsnummer: WO 1999/032146

(56) Entgegenhaltungen:
- EP-A- 0 877 086
- US-A- 4 500 512
- JENNINGS A. D. ET AL.: "Analysis of a yellow fever virus isolated from a fatal case of vaccine-associated human encephalitis." JOURNAL OF INFECTIOUS DISEASES, Bd. 169, Nr. 3, März 1994, Seiten 512-518, XP002103237
- BARRETT A. D. ET AL.: "Comparison of neurovirulence of different strains of yellow fever virus in mice." JOURNAL OF GENERAL VIROLOGY, Bd. 67, Nr. 4, April 1986, Seiten 631-7, XP002103238
- DUNSTER L. M. ET AL.: "Attenuation of virulence of flaviviruses following passage in HeLa cells." JOURNAL OF GENERAL VIROLOGY, Bd. 71, Nr. 3, März 1990, Seiten 601-607, XP002103239
- Tropical Medicine and International Health,Bd.4, Dezember 1999, Seiten: 1-5

## Beschreibung

Die Erfindung betrifft eine Arzneimittelzubereitung zur intra-nasalen Impfung gegen Gelbfieber.

In vielen Gebieten Afrikas und Südamerikas stellt Gelbfieber ein ernsthaftes Gesundheitsproblem dar. Seit der Einführung des lebenden, abgeschwächten 1 7D-Impfstoffstammes gegen die Gelbfieber-Virusinfektion von Theiler im Jahre 1937 steht ein sehr wirksamer Impfstoff gegen diese Infektion zur Verfügung, welcher praktisch keine Nebenwirkungen aufweist (Theiler et al., J. Ex. Med. 65 (1937) 787-800). Die vorbeugende Immunisierung mit diesem Wirkstoff ist sehr effektiv, da die Immunität ein Leben lang anzuhalten scheint. Aufgrund unvollständiger Impfung der Bevölkerung mit 17D in endemischen Gebieten traten und treten jedoch immer wieder Gelbfieberepidemien auf (Robertson et al., JAMA 276, Nr. 14 (1996) 1157-1162). Dies hängt vor allem damit zusammen, dass die im Stand der Technik verwendete subkutane Gelbfieber-Impfung mit einem hohen Aufwand verbunden ist, und somit in vielen Ländern oftmals nicht flächendeckend eingesetzt werden kann.

A.D. Jennings et al., J. Infect. Diseases 169 (1994) 512-518 beschreibt die Analyse einer tödlichen Mutante des Gelbfieberviruses, nämlich der neuroinvasiven Mutante P-16065. Dazu wurden Mäuse intra-zerebral und intra-nasal mit verschiedenen Gelbfieberviren inokuliert, während Untersuchungen an Affen mittels intra-zerebraler Inokulation durchgeführt wurden.

A.D.T. Barrett et al., J. gen. Virol. 67 (1986) 631-637 beschreibt Untersuchungen zur Virulenz von verschiedenen Gelbfiebervirusstämmen an Mäusen, wobei die Viren über verschiedene Applikationsrouten verabreicht wurden.

EP 0 877 086 A2 beschreibt rekombinante Gelbfiebervirus-Impfstoffe, die aus infektiöser cDNA gewonnen wurden.

Eine Aufgabe der Erfindung war es deshalb, eine Arzneimittelzubereitung zur Impfung gegen Gelbfieber bereitzustellen, die in einer einfacheren Anwendungsform als durch subkutane Injektion verwendet werden kann.

Diese Aufgabe wird erfindungsgemäß durch eine Arzneimittelzubereitung zur intra-nasalen Impfung gegen Gelbfieber gelöst.

Hierbei wurde festgestellt, dass bei intra-nasaler Verabreichung eines Impfstoffes gegen Gelbfieber eine wirksame Immunantwort erzielt werden kann, die einen zuverlässigen Impfschutz erwarten lässt.

Durch Verabreichung eines Wirkstoffs gegen Gelbfieber durch die Nase kann ein zuverlässiger Impfschutz erhalten werden, der dem durch subkutane Impfung zu erzielenden Impfschutz nicht nachsteht. Im Gegensatz dazu wird bei anderen Verabreichungswegen, wie etwa oral oder gastro-intestinal, keine Immunantwort erzielt, die einen zuverlässigen Impfschutz erwarten lässt. Durch eine intra-nasale Impfung ist eine ökonomische Verabreichung möglich, die eine allgemeine und einfach durchzuführende Impfung ermöglicht und damit insbesondere für Massenimmunisierungen geeignet ist.

Bevorzugt umfasst die erfindungsgemäße Arzneimittelzubereitung als Wirkstoff den Gelbfieber-Impfstoffstamm 17D, insbesondere einen lebenden, abgeschwächten 17D Gelbfieber-Impfstoff, entsprechend den Bestimmungen der Weltgesundheitsorganisation (Barry et al., "Requirements for Yellow Fever Vaccine", WHO Technical Report Series, Nr. 594 (1976) 34-35). Eine einzelne Impfdosis enthält bevorzugt mindestens 10³ plaquebildende Einheiten (PFU, Plaque Forming Units).

Der Wirkstoff wird zur intra-nasalen Verabreichung bevorzugt in ein geeignetes Lösungsmittel eingebracht. Beispiele für geeignete Lösungsmittel sind physiologische Salzlösungen, wie etwa eine 0,9 %-ige Natriumchloridlösung. Bevorzugt wird der Wirkstoff unmittelbar vor der Verwendung in der Salzlösung, z.B. Kochsalzlösung, aufgelöst. Weitere geeignete Lösungsmittel sind destilliertes Wasser und schweres Wasser (D₂O), mit denen eine hervorragende Stabilisierung des Impfstoffes erreicht werden kann. Weiterhin kann die erfindungsgemäße Arzneimittelzubereitung noch weitere pharmazeutisch geeignete Lösungsmittel und/oder Hilfsstoffe umfassen. Das Lösungsmittel wird insbesondere zur Auflösung und Applikation von gefriergetrocknetem 17D Impfstoff eingesetzt.

Ein weiterer Gegenstand der Erfindung ist die Verwendung einer Arzneimittelzubereitung, die als Wirkstoff den Gelbfieber-Impfstoffstamm 17D umfasst, für die intra-nasale Impfung gegen Gelbfieber. Bevorzugt wird ein lebender, abgeschwächter 17D-Impfstoffstamm verwendet. Überraschenderweise wurde festgestellt, daß bei Verabreichung des Wirkstoffs durch die Nase eine zuverlässige Immunantowrt erzielt werden kann, die einen zuverlässigen Impfschutz erwarten lässt.

Weiterhin umfasst die Erfindung die Verwendung eines Wirkstoffes, umfassend den Gelbfieber-Impfstoffstamm 17D zur Herstellung eines Arzneimittels für die intra-nasale Impfung gegen Gelbfieber.

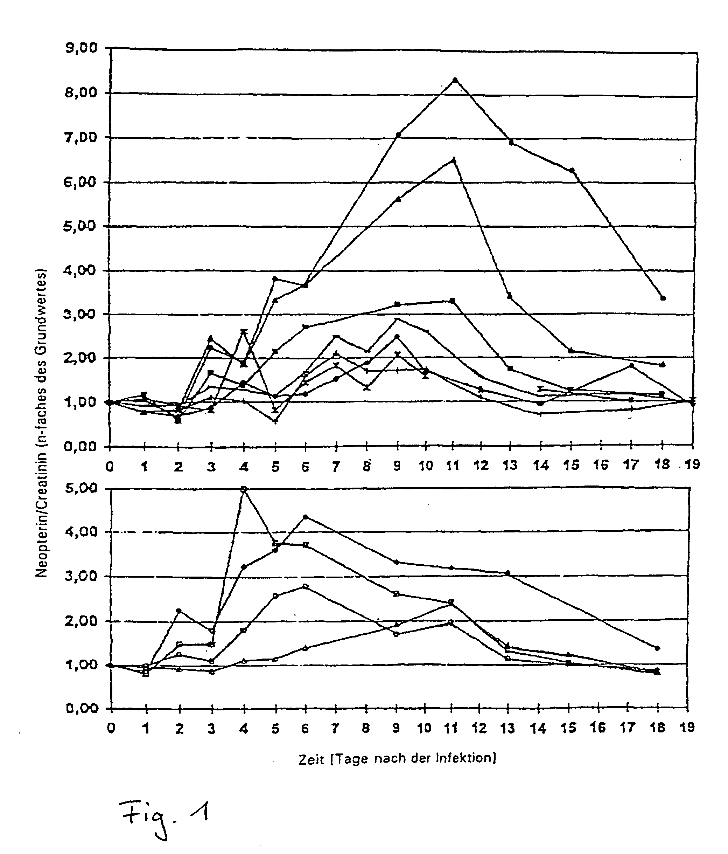

Die Erfindung wird durch die folgenden Beispiele und durch Figur 1 weiter erläutert.

Figur 1 zeigt Neopterin/Creatinin-Werte von intra-nasal, peroral und subkutan geimpften Affen, dargestellt als n-faches des Grundwertes (Wert vor der Impfung). Ausgefüllte Symbole bezeichnen bei der Virusisolierung positive Affen, die intra-nasal oder peroral geimpft wurden (+ = 8488, ■ = 1655, Δ = 1635, ⇑ = 1637, ◇ = 1863, _ = 1889, × = 1891; die Zahlen geben jeweils die Kennummer des Versuchstieres an). Die offenen Symbole bezeichnen die vier, zur Kontrolle subkutan immunisierten Affen ( = 8482, ◇ = 8493, Δ = 8494, ○ = 8495), deren Werte im unteren Diagramm gezeigt sind.

### Beispiel 1

### Impfung von Rhesus-Affen (Macaca mulatta)

Vier Gruppen von 4 bis 6 Rhesus-Affen wurden jeweils mit dem lebenden abgeschwächten Gelbfieber-Impfstoffstamm 17D geimpft. Als Tiermodell für die Immunisierung von Menschen mit einem Gelbfieber-Impfstoff wurden junge bzw. ausgewachsene Rhesus-Affen (Macaca mulatta) mit einem Körpergewicht zwischen 3 und 7 kg verwendet. Aufgrund ihrer größeren Nasenflügel wurden sechs ausgewachsene Affen für die intra-nasale Impfstoffverabreichung ausgewählt, so daß herkömmliche Spray-Vorrichtungen verwendet werden konnten. Die anderen drei Gruppen umfassten jeweils 4 junge Affen. Alle Affen wurden in separaten Käfigen gehalten. Blutproben wurden unter Ketamin-Anästhesie eine Woche vor der Impfung und am 2, 4, 6, 14, 28 und etwa 130 Tag nach der Impfung (p.v.) genommen. Alle Seren wurden in Aliquots bei -70 °C bis zur Verwendung gelagert. Zur Messung des Neopterin-Gehalts wurden zwischen dem 1 und 10 Tag nach der Impfung täglich und bis zum 19 Tag in 2-tägigen Intervallen Urinproben gesammelt.

Als Wirkstoff wurde ein lebender, abgeschwächter 17D Gelbfieber-Impfstoff (Lot Nr.: 142/94/1) verwendet, der am Robert-Koch-Institut, Berlin, Deutschland gemäß den Bestimmungen der WHO (Barry et al., Requirements for Yellow Fever Vaccine, WHO Technical Report Series, No. 594 (1976) 34-35) hergestellt worden war. Eine einzelne Impfdosis enthielt mindestens 6,8 x 10⁴ plaquebildende Einheiten (PFU) und wurde in einem entsprechenden Volumen einer 0,9 %-igen Natriumchloridlösung unmittelbar vor der Verwendung gelöst.

Einer ersten Gruppe von sechs Tieren wurde der Impfstoff unter Verwendung einer Spray-Vorrichtung in jeden Nasenflügel verabreicht.

Einer zweiten Gruppe wurde eine 17D Impfdosis durch orale Spray-Applikation mit einer Spray-Vorrichtung (130 µl pro Hub) direkt in den Rachen eingebracht. In diesem Fall wurden drei Hübe entsprechend einer menschlichen Dosis eingebracht. Dieser Verabreichungsweg wird hierin als peroraler Weg bezeichnet.

Einer dritten Gruppe von Affen wurde der 17D Impfstoff eingekapselt über den Gastro-Intestinalweg verabreicht. Für diesen Zweck wurde der Impfstoff in Gelatinekapseln gefüllt und mit einem säurestabilen Überzug aus Cellulose beschichtet. Die Kapseln wurden hinsichtlich ihrer Säurestabilität durch Inkubation mit 0,1 N HCl für 24 Stunden bei 37 °C getestet. Um zu verhindern, dass die Tiere die Kapseln durch Beißen beschädigten, wurden die Kapseln direkt unter Verwendung einer Sonde geeigneter Größe in den Magen eingebracht.

Eine vierte Gruppe, bestehend aus vier weiteren Affen, diente als positive Kontrolle. Diesen Tieren wurde der 17D Impfstoff, gelöst in 0,5 ml 0,9 %iger Natriumchloridlösung subkutan verabreicht.

Nach der Impfung wurde der Verlauf der durch den 17D Impfstoffstamm induzierten Viremia durch den quantitativen Plaque-Assay aus dem Serum verfolgt, das am vierten und sechsten Tag nach der Impfung abgenommen wurde. Der Assay wurde gemäß den Vorschriften von De Madrid et al., A simple micro-culture method for the study of group B arboviruses, Bulletin of the World Health Organization 40, (1969) 113-121 durchgeführt. Die Ergebnisse sind in Tabelle 1 gezeigt. Die Entwicklung von Gelbfiebervirus spezifischen Antikörpern wurdedurch einen Immunofluoreszenz-Assay (IFA) und durch einen Neutralisationsassay, wie unten beschrieben, bestimmt.

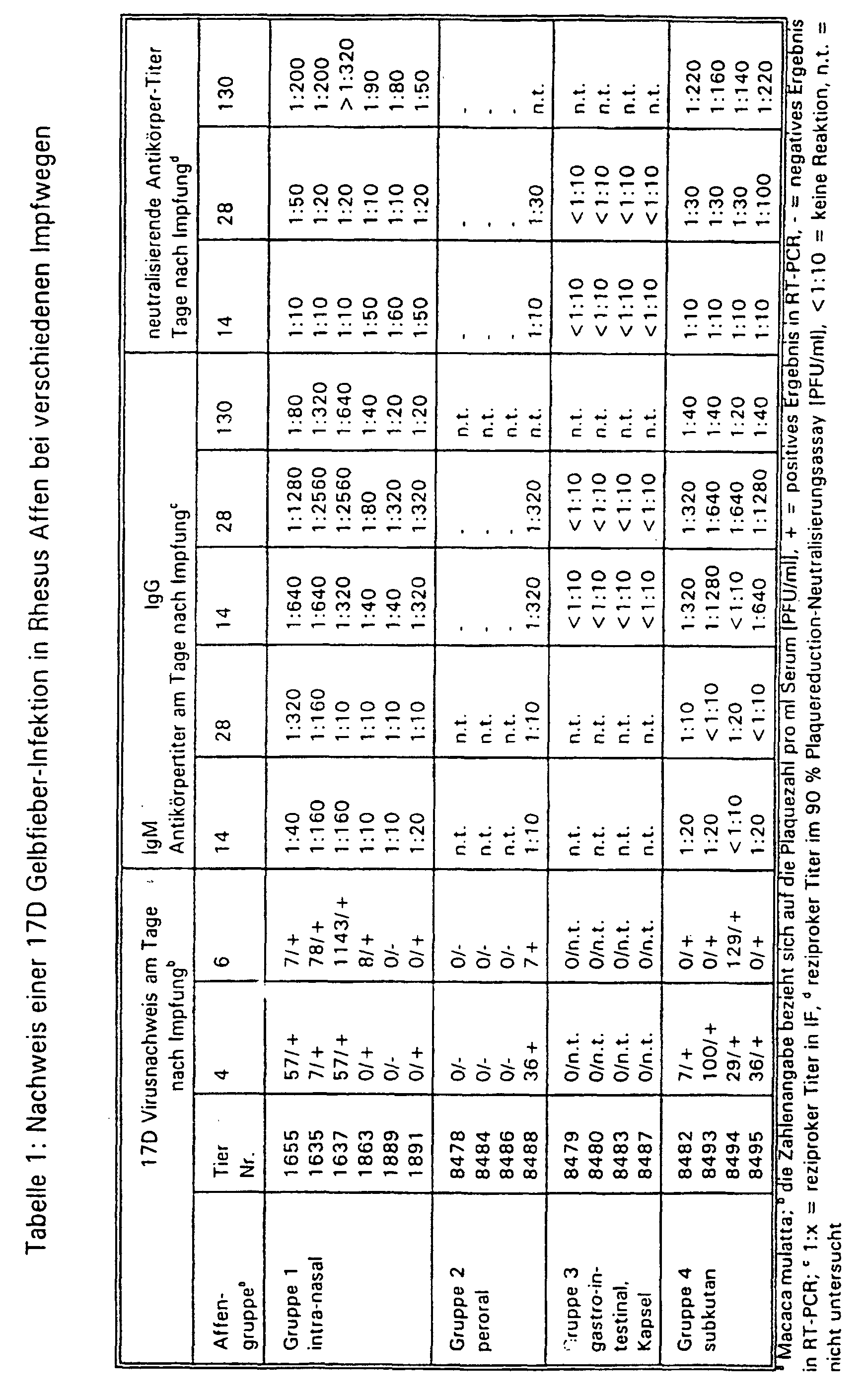

Die Gelbfieber-Impfung wurde von allen Affen insgesamt gut aufgenommen und es wurden keine Nebenwirkungen beobachtet. Bei intra-nasaler Verabreichung des Impfstoffes wurden alle sechs Affen produktiv infiziert, wie durch den Nachweis der Virus-spezifischen Antikörper gezeigt wurde. Im Gegensatz dazu zeigte bei Verwendung der peroralen Impfroute nur eines von vier Tieren (das Tier Nr. 8488) Anzeichen einer Infektion mit 17D. Bei der gastro-intestinalen Impfstoffverabreichung entwickelte keines der vier Tiere Zeichen einer Gelbfieberinfektion. Die vier Affen der positiven Kontrollgruppe, denen der Impfstoffstamm subkutan injiziert worden war, zeigten hingegen eine klare humorale Immunantwort gegen das Gelbfiebervirus. In fast allen Tieren, die virusspezifische Antikörper gegen 17D bildeten und dadurch eine erfolgreiche Impfung anzeigten, wurde am 4 und 6 Tag nach der Impfung das 1 7D Virus durch einen Plaque-Assay isoliert oder die virale RNA wurde nachgewiesen.

Für den RT-PCR wurde RNA durch eine modifizierte Form der früher bereits von Boom et al. (Boom et al., J. Clin. Microbiol. 28 (1990) 495-503) beschriebenen Vorgehensweise extrahiert. Hierzu wurden 50 µl des Serums mit einem Guanidinisothiocyanat-Lysepuffer und mit größenfraktionierten Siliciumoxidpartikeln gemischt. Nach einer 10-minütigen Inkubation bei Raumtemperatur wurde die Lösung gemischt und zentrifugiert. Das Siliciumoxid-Nukleinsäurepellet wurde zweimal mit 1 ml Waschpuffer, zweimal mit 1 ml 70 %-igem Ethanol und einmal mit 1 ml Aceton gewaschen. Das Pellet wurde in einen Wärmeblock trocknen gelassen. Die Nukleinsäuren wurden durch Zugabe von 70 µl eines TE-Puffers zu dem Pellet eluiert. Die Suspension wurde gründlich gemischt und wiederum inkubiert. Nach einer Zentrifugation wurden 50 µl des die RNA enthaltenden Überstandes extrahiert. Für den ersten Durchgang einer nested PCR wurden bereits veröffentlichte Flaviviurs-spezifische Primer verwendet, gefolgt von einer nested PCR mit einem neu entworfenen, spezifischen, Primer (CAC Aag TgA ATT Tgg Cg), (Eldadah et al., J. Med. Virol. 33 (1991) 260-267).

Die meisten der gegenwärtig verwendeten Gelbfieber-Impfstoffe induzieren eine starke T-Helferzellen abhängige Neutralisierungsantikörper-Antwort nach einer Impfung mit lebenden 17D Viren, um mögliche Wildtyp-Gelbfieber-Infektionen zu bekämpfen. Diese Tatsache wurde von mehreren Gruppen untersucht und nachgewiesen (siehe z.B. Smithburn et al., Am. J. Trop. Med. 25 (1945) 217-223; Hill et al. J. General Virol. 73 (1992) 1115-1123; Monath, Yellow fever vaccines: the success of empiricism, pitfalls of application and transition to molecular vaccinology, Hrsg. S. Plotkin & B. Fantini, Vaccinia, vaccination and vaccinology: Jenner, Pasteur and their successors, Elsevier, Paris, 1996, Seiten 157-182). Deshalb wurde die Ausbildung von Bindungs- und Neutralisierung- von Antikörpern gegen den 17D Impfstoffstamm untersucht.

Der Immunoffuoreszenztest für 17D-spezifische IgM und IgG wurde mit 1 7D infizierten PS-Zellen durchgeführt, die dem Stand der Technik entsprechen. Es werden mit 17D infizierte VeroB4-Zellen auf Objektträger aufgebracht und mit Azeton fixiert. Die fixierten Zellen werden mit einer Serumverdünnung (1:10, 1:20, etc.) im Phosphatpuffer pH 7,2 überschichtet und eine Stunde bei 37 °C inkubiert. Nicht gebundene Antikörper werden durch dreimaliges Waschen der Objektträger beseitigt. Hiernach erfolgt die spezifische Markierung mit dem Fluoreszenzfarbstoff gekoppelten Nachweisantikörper (z.B. anti-human IgG FITC). Nach drei weiteren Waschschritten wird das Präparat getrocknet und kann unter dem Fluoreszenzmikroskop ausgewertet werden. Stark leuchtende Regionen in den 17D infizierten Zellen sind ein Nachweis für spezifische Antikörper, die gegen 17D Strukturproteine gerichtet sind.

Nicht infizierte PS-Zellen wurden als negative Kontrollen verwendet. Vor der Impfung wurden in den Tieren keine spezifischen Anti-17D-IgM oder Anti-17D-IgG-Antikörper nachgewiesen. Innerhalb von zwei Wochen nach der 17D Gelbfieber-Impfung konnten hohe Konzentrationen an spezifischen Anti-17D-IgM-Antikörper in erfolgreich geimpften Tieren mit Titern zwischen 1:10 und 1:160 nachgewiesen werden, die entweder konstant blieben oder innerhalb von 2 Wochen abnahmen. Zusätzlich konnte die Gegenwart von spezifischen Anti-17D-IgG-Antikörpern für diese Tiere am 14 und 28 Tag nach der Impfung gezeigt werden, mit maximalen Titern zwischen 1:80 und 1:2560. Sechs Monate nach der Immunisierung hatten alle erfolgreich geimpften Tiere beständige IgG-Titer zwischen 1:20 und 1:640.

Weiterhin wurde Neopterin, ein von Monozyten/Makrophagen, insbesondere nach einer viralen Stimulation freigesetztes Molekül, im Serum und im Urin von allen Tieren gemäß der Vorgehensweise von Huber et al. (Huber et al., J. Exp. Med. 160, (1994) 310-316) analysiert. Für den Nachweis von Neopterin im Serum wurde ein kommerziell erhältlicher Assay (Brahms Diagnostica GmbH, Berlin, Deutschland) gemäß den Instruktionen des Herstellers verwendet. Das Neopterin-Niveau im Urin wurde wie bereits früher beschrieben analysiert (Fendrich et al, AIDS 3 (1989) 305-307). Um die Neopterin-Werte an die Unterschiede im spezifischen Uringewicht anzupassen, wurden die Werte auf die Creatinin-Konzentrationen bezogen. Aufgrund der Unterschiede der einzelnen Neopterin-Grundniveaus wurden die nach der Impfung erhaltenen Konzentrationen zudem auf die Werte vor der Impfung bezogen und als n-faches der jeweiligen Grundwerte ausgedrückt. Parallel zur Viremia konnte ein deutliches Ansteigen von Neopterin bei allen 17D Virus-infizierten Tieren gezeigt werden (vgl. Figur 1). Die in drei intra-nasal geimpften Affen (Nr. 1655, 1636 und 1637) bestimmten Serumneopertinkonzentrationen zeigten Peakwerte am 6. Tag nach Impfung mit Konzentrationen zwischen 8 und 12,5 nm/ml. Nach diesem Peak nahm das Serumneopterin langsam ab. Die am 18./19. Tag nach der Impfung gemessenen Niveaus waren ähnlich den Niveaus vor der Impfung. Bei den subkutan geimpften Tieren konnte ein deutliches Ansteigen des Serumneopterins am 2. Tag nach der Impfung beobachtet werden, welches bis zum 6. Tag für die Tiere 8482 und 8495 mit einem Gehalt bis zu 16 nm/ml dauerte. Allgemein kann festgehalten werden, dass die Neopteringehalte im Blut und Serum miteinander korrespondieren, wobei im zeitlichen Verlauf nur sehr geringe Unterschiede auftreten.

Der Neutralisationsplaquereduktionsassay wurde wie früher beschrieben an PS-Zellen durchgeführt (De Madrid et al., A simple micro-culture method for the study of group B arboviruses, Bulletin of the World Health Organization 40 (1969) 113-121). Kurz zusammengefasst wurden zweifache Verdünnungen der Affenseren im Bereich von 1:20 bis 1:320 mit Dosiseinheiten von 100 plaquebildenden Einheiten Kulturinfektivität eines 17D Virus-Referenzpräparats (Lot. Nr. 354/1 des Anmelders) gemischt. Nach 1 Stunde Inkubation bei 37 °C wurden 0,2 ml dieses Gemisches dem gleichen Volumen einer PS-Zellsuspension zugegeben, die 6 x 10⁵ Zellen/ml enthielt. Eine mit 3 % FCS (fötales Kälberserum) ergänzte 1,6 %-ige CMC/L-15 Lösung (BDH Chemicals Ltd., U.K.) wurde nach 4 Stunden Inkubation überschichtet. Die Kulturen wurden für 5 Tage bei 37 °C gehalten. Danach wurden die Zellen mit PBS gewaschen und mit einer 10 %-igen Formaldehydlösung für 10 Minuten fixiert. Naphthalinschwarz wurde zum Anfärben der Zellschichten verwendet, die Plaques wurden gezählt und der Reciproktiter von 90 % Neutralisation wurde berechnet. Alle auf die Impfung ansprechenden Tiere entwickelten einen Neutralisationstiter im Bereich von 1:10 bis 1:100 innerhalb von 4 Wochen nach der Impfung und hohe Titer zwischen 1:50 und 1:320, insbesondere zwischen 1:80 und 1:320 nach 4 Monaten. Auf diese Weise konnte auch
mit intra-nasaler Verabreichung des Impfstoffes ein zuverlässiger und lang andauernder Impfschutz erhalten werden. Wie in menschlichen Impfversuchen gezeigt, bestehen Titer von neutralisierenden Antikörpern mindestens 10 Jahre nach einer ersten Impfung (Rosenzweig et al., Am. J. Trop. Med. Hyg. 12 (1963) 230-235; Poland et al., Persistance of neutralizing antibody 30-35 years after immunization with 17D yellow fever vaccine, Bulletin of the World Health Organization 59(6) (1981) 895-900).

Im Gegensatz zur intra-nasalen Verabreichung zeigte bei der peroralen Impfstoffverabreichung nur eines von vier Tieren eine deutliche Antwort gegenüber 17D. Diese Route ist somit nicht für eine 17D-Impfung geeignet. Die intra-nasale Spray-Verabreichung ergab hingegen überraschenderweise eine erfolgreiche Impfung für alle sechs untersuchten Affen. Auch die gastro-intestinale Verabreichung eines eingekapselten 17D Impfstoffes führte zu keiner Infektion der Testtiere, sodass auch durch diesen Verabreichungsweg kein Impfschutz erzielt werden kann.

Zusammenfassend kann festgehalten werden, dass die intra-nasale Verabreichung eines lebenden, abgeschwächten 17D Impfstoffes wirksam eine schützende Immunität induzieren kann. Die intra-nasale Anwendung eines 17D Impfstoffes stellt somit eine geeignete und ökonomisch interessante Alternative für Massenimpfungen in betroffenen Ländern dar, insbesondere da Injektionen mit Impfpistolen mit einem hohen Risiko der Hepatitis B Übertragung verbunden sind (Abb et al., J.Infect. Diseases 144 Nr. 2 (1981) 179; Brink et al., J. Med. Microbiol. 20 (1985) 393-397, Shah et al., Morbidity and Mortality Weekly Report 35, Nr. 23 (1986), 373-376).

## Patentansprüche

1. Verwendung eines Wirkstoffes, umfassend den Gelbfieber-Impfstoffstamm 17D zur Herstellung eines Arzneimittels für die intra-nasale Impfung von Menschen gegen Gelbfieber.

2. Verwendung nach Anspruch 1,
**dadurch gekennzeichnet,**
**dass** der Wirkstoff in einer physiologischen Salzlösung gelöst vorliegt.

3. Verwendung nach einem der vorhergehenden Ansprüche,
**dadurch gekennzeichnet,**
**dass** das Arzneimittel weiterhin pharmazeutisch geeignete Hilfs- oder/und Trägerstoffe umfasst.

## Claims

1. Use of an active substance comprising the yellow fever vaccine strain 17D for producing a medicament for intranasal vaccination of humans against yellow fever.

2. Use according to Claim 1, **characterized in that** the active substance is dissolved in a physiological salt solution.

3. Use according to either of the preceding claims, **characterized in that** the medicament additionally comprises pharmaceutically suitable excipients or/and carriers.

## Revendications

1. Utilisation d'un agent actif renfermant la souche de vaccin 17D contre la fièvre jaune pour fabriquer un médicament destiné à la vaccination intra-nasale de l'homme.

2. Utilisation selon la revendication 1,
**caractérisée en ce que**
l'agent actif se présente dissous dans une solution saline physiologique.

3. Utilisation selon l'une quelconque des revendications précédentes,
**caractérisée en ce que**
le médicament renferme par ailleurs des excipients et/ou des substances supports adaptées au plan pharmaceutique.
